(19) Europäisches Patentamt
European Patent Office
Office européen des brevets

(11) EP 2 866 031 B1

(12) # EUROPEAN PATENT SPECIFICATION

(45) Date of publication and mention of the grant of the patent:
**02.01.2019 Bulletin 2019/01**

(51) Int Cl.:
***G01N 33/52*** *(2006.01)*

(21) Application number: **14190562.0**

(22) Date of filing: **27.10.2014**

(54) **Kit and method for rapidly determining the total antioxidant capacity in whole blood or other biological sample**

Kit und Verfahren zur schnellen Ermittlung der gesamten Antioxidanzienkapazität in Vollblut oder einer anderen biologischen Probe

Kit et procédé permettant de déterminer rapidement la capacité antioxydante totale dans le sang total ou de tout autre échantillon biologique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2013 IT PO20130007**

(43) Date of publication of application:
**29.04.2015 Bulletin 2015/18**

(73) Proprietor: **Solosale S.r.l.**
**50125 Firenze (IT)**

(72) Inventors:
- **Liguri, Gianfranco**
  **50141 Firenze (IT)**
- **Ciuti, Riccardo**
  **50019 Sesto Fiorentino (FI) (IT)**

(74) Representative: **Martini, Riccardo**
**Brema SRL**
**Piazza Enriquez, 22/c**
**47891 Dogana (SM)**

(56) References cited:

- **R Ciuti ET AL: "A NEW ASSAY FOR TOTAL ANTIOXIDANT CAPACITY IN WHOLE BLOOD AND OTHER BIOLOGICAL SAMPLES", Researchgate , 1 June 2014 (2014-06-01), XP055129829, Retrieved from the Internet: URL:http://www.researchgate.net/profile/Gianfranco_Liguri/publication/259450881_A_NEW_ASSAY_FOR_TOTAL_ANTIOXIDANT_CAPACITY_IN_ WHOLE_BLOOD_AND_OTHER_BIOLOGICAL_SAMPLES/file/e0b4952ba9b89bb1fd.pdf?ev=pub_ext_doc_dl&origin=publication_detail&inViewer=true [retrieved on 2014-07-17]**
- **GARRY P J ET AL: "Automated analysis of plasma and whole blood ascorbic acid", CLINICAL BIOCHEMISTRY, ELSEVIER INC, US, CA, vol. 7, no. 1-4, 1 January 1974 (1974-01-01), pages 131-145, XP023600026, ISSN: 0009-9120, DOI: 10.1016/S0009-9120(74)91276-4 [retrieved on 1974-01-01]**
- **OMAYE S T ET AL: "Selected methods for the determination of ascorbic acid in animal cells, tissues, and fluids", METHODS IN ENZYMOLOGY, ACADEMIC PRESS, US, vol. 62, 1 January 1979 (1979-01-01), pages 3-11, XP009179165, ISSN: 0076-6879**
- **HUANG DEIJAN ET AL: "The chemistry behind antioxidant capacity assays", JOURNAL OF AGRICULTURAL AND FOOD CHEMISTRY, AMERICAN CHEMICAL SOCIETY, US, vol. 53, no. 6, 1 March 2005 (2005-03-01), pages 1841-1856, XP002413438, ISSN: 0021-8561, DOI: 10.1021/JF030723C**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a method and a kit for the photometric fast determination of total antioxidant capacity of whole blood or other liquid and/or biological tissue of animal or vegetable origin. The method and the kit are based on the use of a reagent containing a chromogen having the nature of an indophenol derivative. Precisely, the method and the kit of the present invention allow the assessment and monitoring the ability to counteract the oxidative insult caused by free radicals of oxygen and nitrogen species (ROS, NOx) by patients who are in physio-pathological conditions characterized by redox unbalance of their organism due to the excessive production of free radicals and / or lack of molecules with antioxidant action. The invention can also be used to evaluate the antioxidant capacity in individuals exposed to ionizing radiation or during aging process of an organism and to assess the oxidative stress caused by exercise in athletes.

### Background Art

In optimal physiological conditions, inside the body a well-balanced equilibrium between oxidizing and reducing processes exists, which is called redox balance. Oxidative stress is a generic term used to define a physiological or pathological situation where oxidative processes exceed the body's antioxidant defenses. Small amounts of Reactive oxygen species (ROS) and nitrogen reactive species, such as hydroxyl radicals, superoxide anion, nitroxide radical and hydrogen peroxide are continuously generated inside the cells of aerobic organisms. The production of energy by means of the transport of mitochondrial electron generates, under normal conditions, significant amounts of free radicals and represents the most productive site of radicals in the body. Despite these reactive molecules have a lifespan in the order of milli- or nano-seconds they are capable, if not properly neutralized, of altering biologically important molecules like lipids, proteins and DNA. Changes affecting these molecules, that are induced by oxidation, can themselves damage and influence many cellular functions causing their dysfunction or even apoptosis. Oxidative stress is associated with various pathological processes and degenerative diseases such as atherosclerosis, diabetes, kidney failure, cancer, inflammatory and neuro-degenerative processes, and, generically, to the aging processes.

Consequently, evaluating the redox balance of an individual is a basic test for his health. The correct strategy is to assess both the level of these oxygen-reactive molecules and the antioxidant capacity of the organism. In the first case the difficulty of accurately quantifying ROS is not surmountable, given their ephemeral life expectancy, unless sophisticated and expensive methodologies, such as electron spin resonance (ESR) are used; alternatively, the molecules which have been oxidated can be determined, but these, belonging to the class of the various lipids, proteins and nucleotides, are individually measurable and represent only a part of the whole oxidative insult. In the second case the determination of total antioxidant capacity allows the evaluation of the effective defense power of the organism. In humans, the substances with antioxidant capacity and capable in various ways and entities of neutralizing ROS are numerous and perform their action in different districts of the organism, both in plasma and at intracellular level. A redox balance evaluation can be carried out using electrochemicals methods, for example by means of electrodes for measuring the redox potential (Oxidation Reduction Potential, ORP). These methods, however, are expensive and are characterized by instability of the signal, long measurement times and poor accuracy, such that their use is not compatible with the common laboratory practice. Even the chemical and biochemical methods, currently available for the determination of total antioxidant capacity values, provide inaccurate or partial values and are unfit for measuring the antioxidants contained within cells and / or because they cannot detect all the chemical antioxidants species present in the samples.

Garry et al. (Clin. Biochem. 7, 131-145, 1974) disclose the use of 2,6-dichloro phenol indophenol (DCPIP or DCIP) for the determination of vitamin C (ascorbic acid) in plasma and whole blood, comprising adding meta-phosphoric acid to a plasma sample, or alternatively lysing blood cell components by diluting whole blood 1.1 with water and freezing the sample. The samples are then reacted with DCIP. All reactants comprise the detergent Brij-35 and do not comprise a chelating agent, a substance capable of reducing the polarity of the solvent or a preservative.

Omaye et al. (Meth. Enzymol. 62, 3-11, 1979) disclose a similar process for the determination of vitamin C in animal cells, tissues and fluids. Meta-phosphate is added in both the cited documents to remove any interfering proteins. Huang et al. (J. Agricult. Food Chem. 53, 1841-1856, 2005) review the assays for determination of antioxidant capacity.

The present invention, based on the use of an appropriate redox indicator, provides analytical results which are similar to but more accurate than those obtained by electrochemical or by analytical methods based on the use of chemical reagents, but without their drawbacks. The described kit, in fact, thanks to the presence of a lysing agent is able to lyse completely tissue cells, and then to measure the total antioxidant capacity of the whole sample, i.e. the overall contribution to the redox equilibrium of the antioxidants present in both intracellular and extracellular compartments. Moreover, thanks to the use of a chromogen with a redox potential that allows the reduction by all the antioxidants present in the biological samples, the kit is capable of determining the total antioxidant capacity, i.e. the

overall contribution to the redox balance from different molecules with antioxidant activity contained in the sample.

Disclosure of Invention

It is an object of the present invention to provide a kit and a method for the determination of Total Antioxidant Capacity (TAC) in whole blood and in other biological samples for the diagnosis of pathological states in which an altered balance between ROS production and antioxidant defenses is documented, such as Alzheimer's disease, Crohn's disease, many cancers, etc., and for the assessment and monitoring of particular physiological states such as pregnancy, physical hyperactivity, stress, senescence, etc.

According to the invention, this object is solved by a kit and a method as defined in claim 1 and 9.

The invention may also be used to estimate the oxidative stress in subjects exposed to ionizing radiation. For this purpose, the kit makes use of an indicator whose value of redox potential is such that the antioxidants contained in the sample cause its rapid reduction to an extent that depends on the redox balance of the organism.

Moreover, since the substances able to counteract the oxidative stress are mostly confined inside the cells, the method and the kit proposed allows measurements of TAC both in biological fluids and lysated cells, in particular on hemolysates. This feature makes the determination of the TAC in a very versatile and easy way, since the blood tissue is easily accessible.

In particular the invention provides a kit suitable for determining the total reducing power in whole blood, other tissues or biological fluids by measuring the reduction of the redox indicator affected by all the reducing substances, each with its own reduction potential, present in the sample. This measure can be usefully performed photometrically since the reduction of the indicator is associated to a discoloration of the same. In fact, the aqueous solution of the indicator used is colored with vivid blue when oxidated and colorless when reduced.

The TAC analysis in whole blood is possible thanks to:

1) the presence of a surfactant with cytolytic function, in the reagent used in the kit, that allows for the rapid lysis of red blood cells and the optimal interaction between the indicator and substances with a reducing action present within blood cells and in plasma (glutathione, uric acid, ascorbate, bilirubin, etc.);

2) the use of a chromogen whose absorption spectrum does not interfere, considering the wave lengths which measurement is made at, with the absorption spectrum of hemoglobin.

These characteristics, which are essential to be able to determine the TAC on whole blood, are advantageous also in the analysis of biological samples of different nature, coming from both human and animal or plant. The test can in fact be used for analyzing any biological fluid, such as whole blood, serum or plasma, urine, saliva, cerebrospinal fluid, tears, semen, vaginal secretions, amniotic liquid, maternal milk and cord blood. Additionally, the kit of the present invention can be used with tissue homogenates, lysed cells, washings bronchial and dialysis fluids, etc. The samples of blood, venous, arterial or capillary, can be collected on any type of test tube (glass or plastic) with any type of anticoagulant. EDTA is still preferable, as it preserves most of the sample from its own oxidation. Other samples that can be analyzed by this kit are, for example, juices or extracts of fruits and vegetables, such as olive oil, and drinks produced by fermentation such as wine, beer, cider, etc.

The kit can also be used to evaluate and monitor the shelf life of blood transfusion bags in the various centers of Blood Bank. It was in fact observed that the oxidative insult grows over time during conservation; therefore, the measurement of the TAC may indicate the appropriate maintenance and function of whole blood, platelets, frozen plasma.

Physical exercise is associated with an increased aerobic and / or anaerobic metabolism that increases the formation of reactive oxygen species substances (ROS). The intense and prolonged physical effort can generate such a level of ROS that the antioxidant defense system of the athlete cannot be sufficient to neutralize it. Both the aerobic and anaerobic physical effort produces excess of free radicals, especially if the athlete is not sufficiently trained and has not undergone an appropriate time of rest after a race. The kit of the invention can therefore be used to assess and monitor the athletic performances of patients engaged with both professional and amateur sports. Persistently abnormal TAC values in an athlete can highlight an "Overreaching" or even "Overtraining" syndrome.

The TAC measurement can be useful to evaluate the progression in the different phases of the atherosclerotic process. A poor defensive power against free radicals may cause the formation, in blood vessels, of the so-called "Foam cells", which are the result of lipid peroxidation and give rise to atheromatous plaques. Atherosclerosis, itself, is a predisposing condition for other cardiovascular diseases, such as myocardial infarction (MI) and stroke. During the process of "ischemia-reperfusion injury", which runs in these acute pathologies, damage occurs by oxidative insult which is caused by the formation of ROS through the activation of xanthine oxidase. So, also the extent of the phenomenon is quantifiable through the measurement of the values of the TAC.

A lack of balance between the production of ROS and an adequate defensive power also occurs in diabetes mellitus. Hyperglycemia and insulin resistance, along with the formation of oxidized protein adducts, such as AGE

(Advanced Glycation End products), are all factors that promote oxidative stress. Even in diabetes mellitus the test object of the present invention may therefore be advantageously used.

The invention is also useful for assessing the body's ability to neutralize ROS produced by exposure to ionizing radiation in cancer patients undergoing radiotherapy and to measure the antioxidant capacity in patients treated with drugs known as inducers of ROS, such as tricyclic antidepressants (TCA), valproic acid (VPA), etc., or in patients intoxicated by drugs of abuse such as ethanol, cocaine, etc.

It is generally agreed in the international scientific community that the Oxidative stress plays a fundamental role in the aging process, through the increasing damage caused in the cellular DNA structure, and through structural alteration of cellular proteins and lipids. The measurement of the TAC with the present method can therefore be used to estimate the stage of biological aging of an individual.

Detailed Description of the Invention

The chromogen used for the TAC determination, the specific analyte for the kit described in the present invention, is an indicator of the redox potential that can oxidize the main reducing substances present in whole blood and other organic samples.

The spectrophotometric measurement relies on the fact that the blue chromogen in hydroalcoholic solution, when transformed in its reduced species is colorless, thanks to the reaction with the antioxidants present in the sample.

The reduction reaction of the chromogen is fast and has an almost linear trend during the first minutes after the addition of the sample.

The reaction is quantified spectrophotometrically by measuring the absorbance of the mixture reaction at 630 nm, the wavelength at which a high extinction of the chromogen is manifested in absence of interference by hemoglobin.

The concentration of antioxidants in the sample can thus be determined by measuring continuously the reaction between these and the chromogen (kinetic mode), or at certain times (fixed-time mode) or by measuring the difference of Absorbance of the mixture between the beginning and the end of the reaction (end-point mode).

The TAC determination with the kit herein described requires the use of a photometer or a spectrophotometer operating in the spectrum of visible light and equipped with a filter or a monochromator for the selection of the wavelength corresponding to 630 +/- 100 nm and can be run in both manual and automatic way.

The chromogen studied and used for the invention is a redox indicator belonging to the family of compounds of indophenol derivatives of general formula:

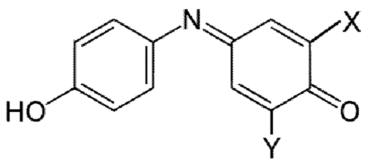

where X is an atom of fluorine, chlorine, bromine or other halogen and Y is a fluorine atom, chlorine, bromine or other halogen. For the purposes of the present invention the halogen derivative of indophenol which has proven to possess the characteristics of reactivity, sensitivity, stability and specificity, that are most suitable for the quantitative determination of total antioxidant capacity of whole blood and other biological samples, was the 2.6-dichlorophenolindophenol (DCPIP). The DCPIP is a redox indicator whose balance between the reduced form and the oxidized changes as a function of redox potential of the solution which is located in (see Fig. I).

Fig. I: redox reaction of 2,6-dichlorophenolindophenol.

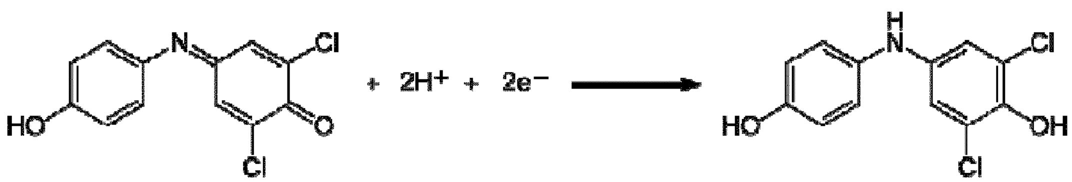

A further reagent that can be used is the 2,6-dibromophenolindophenol, possibly in combination with DCPIP.

Since the redox indicator is also sensible to the pH, it has been decided to remove this dependence by adopting

an effective buffer system. All buffer systems, such as TRIS®, triethanolamine, monohydrogen phosphate/dihydrogen phosphate, etc., which are effective in the pH range from 5.0 to 12.0, can be used in the reagent object of the present invention.

A lysing agent is necessary to cause the disintegration of the cells of the biological sample and make the intracellular substances react with the chromogen.

Among the agents known to those skilled in the art are: - Mechanical disintegration (homogenization);- Ultrasound; - Alternating cycles of freezing/defrosting;- Hypotonic shock; - Solubilization of cell membranes through surfactants or enzymes or other chemical agents, such as sodium dodecyl sulfate, Triton®, hemolysine, phospholipase, etc.

For the implementation of the present invention, the use of hydroxypolyethoxydodecane (THESIT®) has proved surprisingly effective. It is also able to solubilize the cellular constituents, making any pre-treatment procedure of the sample like centrifugation, filtration or dialysis, unnecessary for the subsequent photometric analysis.

For the purpose of complexing bivalent metal ions, potential catalyzers of the oxidation reactions of the chromogen, and therefore to increase its stability, sodium ethylenediaminotetracetate (Na2EDTA) was added as a further component of the reagent.

Furthermore, the formulation of the present invention contains a substance capable of reducing the polarity of the solvent and, therefore, increasing the solubility coefficient of the chromogen.

Such a reducing substance may be constituted by ethyl ether, methanol, ethanol or propanol with a concentration that ranges from 5% to 50% (v/v), preferably by ethanol with a concentration that ranges from 10% to 20% (v/v).

Finally, in order to ensure an adequate thermal stability, for the commercial use of the product resulting from the present invention, the presence of biocides choosen from compounds used as preservatives in diagnostic kits, preferably methylisothiazolone or chloroacetamide, is expected.

The analytical procedure for the TAC determination with the kit that is object of the present invention follows these steps:

- Insertion of the reagent into the photometer cuvette;
- Measurement of the absorbance of the reagent (A0) in order to verify its validity;
- Addition of the sample (C) to the reagent, stirring and measurement of the photometric signal as described below:

    * in the kinetic mode:

        - Reading the absorbance ($A_1$) of the reaction mixture at a delay time T1 and subsequent readings of absorbance ($A_2$ ... .$A_n$), (at a frequency greater than $sec^{-1}$), during a time interval T2;
        - Determination of the slope of the straight line that linearly interpolates the series of readings ($A_2$ ... .$A_n$);

    * in the fixed time mode:

        - Reading the absorbance $A_1$ and $A_2$ of the reaction mixture at a delay time T1, and at a time T2 within the time interval the reaction proceeds with 0 order kinetics;

    * in the end-point mode:

        - Reading the absorbance $A_1$ and $A_2$ of the reaction mixture at a delay time T1 and at a time T2 subsequent to the equilibration time of the reaction (about 1200" at Room Temperature), respectively;

- Repeat the previous steps using a standard solution (St) with a known concentration of the analyte. For this purpose, titrated solutions of reducing substances such as ascorbic acid, uric acid, glutathione, etc., can be usefully used. Particularly preferred, and used as default for the present method, is a solution of reduced glutathione with a concentration of 2 mM.
- Calculation of the TAC by the following equations.

    A) (for fixed-time and end-point methods):

$$TAC\ (mEq / L) = (A_{2C} - A_{1C}) / (A_{2St} - A_{1St}) * C_{St}$$

where $A_{2C}$ and $A_{1C}$, are sample absorbances; $A_{2St}$ and $A_{1St}$, are standard absorbances; $C_{St}$ is the standard concentration expressed in mEq / L.

B) (for Kinetics method):

$$TAC\ (mEq / L) = k_C / k_{St} * C_{St}$$

where $k_C$ is the slope of the straight line that linearly interpolates the readings of sample absorbance; $k_{St}$ is the slope of the straight line that linearly interpolates the readings of standard absorbance; $C_{St}$ is the standard concentration, expressed in mEq/L.

The use of reduced glutathione, as a reference standard for the present kit, has the considerable advantage of relating the values of TAC to the most abundant intracellular antioxidant.

A problem related to the use of reduced glutathione is, however, its tendency to react with oxygen to generate oxidized glutathione.

This parasite reaction is advantageously avoided, in the kit described here, thanks to the presence, as a stabilizer, of mannitol with a concentration that ranges from 10 to 100 mM and the standard conservation in containers sealed in argon atmosphere, nitrogen or other inert gas.

The use of the method and kit of the present invention to assess the state of the body's antioxidant defenses allows the accurate discrimination of subjects with TAC normal value from subjects whose redox balance is not balanced. TAC levels that are measured in individuals who do not suffer from any disease and that prior to the time of withdrawal, have not carried out relevant physical activities and did not intake drugs or food supplements, are considered reference normal values. Individuals belonging to different age groups have different normal values of TAC. Lower normal TAC values can be found in older subjects.

TAC values, measured in the blood sample, that are lower than normal values show insufficient defensive power of the organism. In these cases further investigation through the dosage of individual markers of oxidative stress such as malondialdehyde, the 8-hydroxy-2'-deoxyguanosine (8-OHdG), the fraction of oxidized low density lipoprotein (ox-LDL), etc., is advised, in order to identify the main source of the radical insult and to individually determine the main antioxidant agents such as reduced glutathione (GSH), uric acid, bilirubin and fat- and hydro-soluble vitamins together with the enzymatic activity of the most important enzymes involved in biochemical defense processes against oxidative stress, such as superoxide dismutase (SOD), glutathione peroxidase, glutathione reductase and catalase.

The kit for the analysis of the total antioxidant capacity in a biological sample here described requires the use of a photometer both for professional use, such as spectrophotometers or microplate readers, and for decentralized situations, such as portable photometers, operating at a wavelength between 580 and 700 nm, preferably at 630 nm. Said photometer can be used even by non professionals thanks to the following features:

- The volume of the sample required for the analysis is below 20 microL that, in case of capillary blood, can be taken by finger pricking;
- Quick time of execution of the analysis, comprised between 30" and 1200";
- Possibility of carrying out the analysis with the use of portable photometers or pocket battery powered photometers, without the use of pipettes or other accessories from laboratory analysis;
- Possibility to perform the analysis at room temperature between 10 and 40 °C.

The kit, object of the present invention, provides the TAC value of a biological sample expressed as mEq / L of glutathione (mEqGSH / L), given the predominant abundance of this antioxidant in animal tissues and given the chemical nature of the standard used in the method of the present invention. The kit can be used with fixed time (fixed-time), final time (end-point) or kinetics methods and can be performed using single cuvettes, flowcuvette or microplates.

Examples

Below some possible embodiments of this kit, but not exhaustive of all possible realization forms of the present invention, are described.

Example # 1:

The total antioxidant capacity was determined using the kit object of the present invention, on 149 venous blood samples from hospitalized patients (OSP), suffering from degenerative diseases, and on 37 samples of venous blood from subjects of control (CTR) of equivalent age, using the "fixed-time" method and the following reagents.

Reagent:

- Ethanol 17.7% (v / v)
- 75 mM phosphate buffer pH 8.8
- 2,6- dichlorophenolindophenol 0.08 mM
- Hydroxypolyethoxydodecane (THESIT®) 0.3% (v / v)
- $Na_2EDTA$ 5 mM
- Stabilizing biocide

Standard:

- 2 mM GSH
- Phosphate buffer 50 mM, pH 6.5
- Mannitol 50 mM
- $Na_2EDTA$ 1 mM
- Biocidal Stabilizer

Both the reagent and the standard have proved to be usable for a period greater than 18 months when stored at 4 °C.

The analysis, carried out using a spectrophotometer VIVA (Siemens AG) operating at wavelength of 630 nm, at 37 °C, using as analytical parameters, values of: T1 = 15"; T2 = 45"; Sample / Reagent ratio = 10 L: 1000 μL, provided the following results:

$$TAC_{OSP} = 2.21 \pm 0.31 \ mEq_{GSH}/L$$

$$TAC_{CRT} = 3.15 \pm 0.25 \ mEq_{GSH}/L$$

The two values differs very significantly (p <0.001).

In the same samples the concentration of intra-erythrocytary GSH was determined spectrophotometrically according to Akerboom et al. (Akerboom, T.P., and Sies, H., "Assay of glutathione, glutathione disulfide, and glutathione mixed disulfides in biological samples. Methods Enzymol. ", 77, 373-382 - 1981) and the following results have been obtained:

$$GSH_{OSP} = 1.55 \pm 0.21 \ mEq/L$$

$$GSH_{CRT} = 2.23 \pm 0.22 \ mEq/L$$

The obtained values of GSH in the two groups correlate very significantly (r = 0.98) with the respective values of the TAC, determined with the use of the kit, object of the invention.

Using the solutions of the reagent and of the standard, described in this example, a change over time of the absorbance of the reagent caused by the reaction of the chromogen with glutathione was registered. In Fig. II the respective kinetics are shown.

Fig. II - Kinetics of the reaction of the reagent.

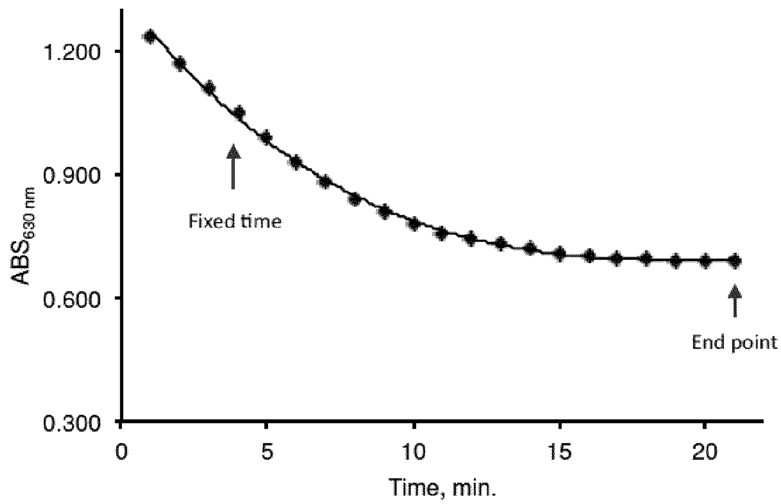


Using the same reagents, the calibration curve shown in Fig. III was also obtained, which demonstrates the kit object of the present invention grants high linearity and accuracy.

Fig III - Calibration curve of the reagent

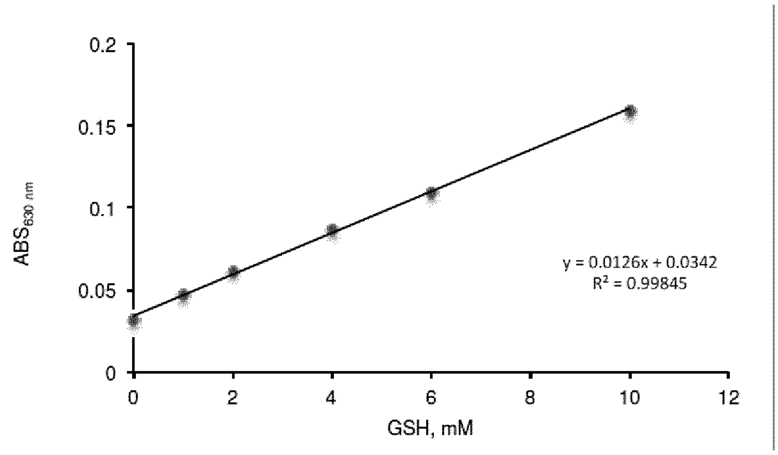


Example # 2:

The kit, object of the present invention, was used to determine the total antioxidant capacity of capillary blood obtained by the fingerpricking of N = 20 athletes before (PRE) and after (POST) a byke race of 3 h, with the average energy consumption of approximately 450 kcal / h, using the "kinetic" method and the following reagents:

Reagent 1

- 25 mM phosphate buffer, pH 8.8
- 0.3% (w/v) Hydroxypolyethoxydodecane (THESIT®)

Reagent 2

- 15.0% (v / v) Ethanol
- 50 mM Buffer TRIS pH 8.8
- 0.1 mM 2,6- Dichlorophenolindophenol
- 0.3% (w/v) Hydroxypolyethoxydodecane (THESIT®)
- 5 mM $Na_2EDTA$

Standard

- 2 mM GSH
- 50 mM phosphate buffer, pH 6.5
- 50 mM Mannitol
- 1 mM $Na_2EDTA$

The blood samples were pre-lysed by mixing 1:3 (v/v) with Reagent 1. The analysis, carried out at room temperature using a microplate reader Biochrom EZ-Read operating at 630 nm and with the analytical parameters T1 = 15", T2 = 75", gave the following results:

$$TAC_{ANTE} = 3.45 \pm 0.24 \text{ mEq}_{GSH}/L$$

$$TAC_{POST} = 2.95 \pm 0.18 \text{ mEq}_{GSH}/L$$

The determination of the intra-erythrocytic concentration of GSH with a spectrophotometer according Akerboom et al. (Akerboom, T.P., and Sies, H., Assay of glutathione, glutathione disulfide, and glutathione mixed disulfides in biological samples. Methods Enzymol., 77, 373-382 - 1981) was carried out on the same samples.

The levels of GSH obtained in the two groups correlate very significantly (p <0001) with the respective values of the TAC determined with the kit described:

$$GSH_{ANTE}\ 20 = 2.38 \pm 0.18 \text{ mEq/L}$$

$$GSH_{POST} = 2.05 \pm 0.14 \text{ mEq/L}$$

Example # 3:

The present kit was used to determine the total antioxidant capacity of 20 extracts of the fruit of *Citrus medica* (CITR) and of 30 fresh juices of the fruit of *Vitis vinifera* (VITIS) with the end-point method and the use of the following reagents:

Reagent

- 15% (v/v) Ethanol
- 50 mM Buffer TRIS pH 8.8
- 0.08 mM 2,6-Dichlorophenolindophenol
- 0.3 % (w/v) Hydroxypolyethoxydodecane (THESIT®)
- 5 mM $Na_2EDTA$

Standard

- 2 mM GSH

- 50 mM Phosphate buffer, pH 6.5
- 50 mM Mannitol
- 1 mM $Na_2EDTA$

The analysis, carried out at room temperature on cuvettes with optical path = 1 cm using a portable photometer equipped with interference filter operating at 630 nm, and analytical parameters T1 = 15", T2 = 1200", gave the following results:

$$TAC_{CITR}\ 10 = 2.40 \pm 0.17\ mEq_{GSH}/L$$

$$TAC_{VITIS} = 13.3 \pm 0.72\ mEq_{GSH}/L$$

The determination of the concentration of ascorbic acid (ASC) by spectrophotometry with the kit FRASC (Sigma-Aldrich Ascorbic Acid Assay Kit II, Catalog Number MAK075) was carried out on the same samples.
The levels of TAC obtained in the two groups of samples correlate very significantly ($r_{CITR}$ = 0.980; $r_{VITIS}$ = 0.975) with the respective concentration values of ascorbic acid:

$$ASC_{CITR} = 480 \pm 12.5\ mg/L$$

$$ASC_{VITIS} = 3200 \pm 80.5\ mg/L$$

## Claims

1. Kit for the photometric determination of the total antioxidant capacity inside and outside cells of whole blood or other biological sample, comprising:

   - A reagent containing:

     a) a chromogen sensitive to the electrochemical potential of the sample, belonging to the family of indophenols, having the general formula:

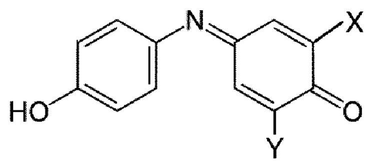

     in which the groups X and Y are halogen atoms,
     b) a buffer system, to maintain a constant pH value between 8.8 and 9;
     c) a surfactant with cytolytic activity consisting of hydroxypolyethoxydodecane;
     d) a chelating agent;
     e) a substance able to reduce the polarity of the solvent;
     f) a preservative agent;

   - A standard containing:

     g) a buffer system, to maintain a constant pH value;
     h) a reducing substance;
     i) a chelating agent;
     1) a preservative agent.

2. Kit according to claim 1, wherein the chromogen (a) is 2,6-dichlorophenolindophenol or 2,6-dibromophenolindophenol or a combination of them.

**3.** Kit according to claim 1 or 2, wherein the standard comprises a reducing substance (h) consisting of reduced glutathione at a concentration ranging from 0.1 to 10 mM, preferably at a concentration of 2 mM, and a stabilizing agent consisting of mannitol.

**4.** Kit according to claim 1, 2 or 3, wherein the buffer system in the reagent is a solution containing $H_2PO_4^-/HPO_4^{2-}$ ions whose concentration is comprised between 5 and 200 mM, preferably between 50 and 100 mM.

**5.** Kit according to claim 1, 2 or 3, wherein the buffer system in the standard is a solution containing $H_2PO_4^-/HPO_4^{2-}$ ions whose concentration is comprised between 5 and 200 mM, preferably between 50 and 100 mM and having a pH between 5.0 and 6.5.

**6.** Kit according to any one of the preceding claims, wherein the chelating agent is sodium ethylenediaminetetraacetic acid ($Na_2EDTA$).

**7.** Kit according to any one of the preceding claims, wherein the reagent comprises a substance (e) able to reduce the polarity of the solvent, such substance being ethyl ether, methanol, propanol or ethanol at a concentration ranging between 5% and 50% (v/v), preferably ethanol between 10% and 20% (v/v).

**8.** Kit according to any one of the preceding claims, wherein the preservative agent is a biocide substance, chosen from any compound used as preservatives in diagnostic kits, preferably methylthioazolinone or chloroacetamide.

**9.** Method for determination of total antioxidant capacity (TAC) inside and outside cells, of whole blood or other biological sample, by using a kit in accordance to claims 1 to 8 and a photometer, comprising the steps of:

- Filling the photometer cuvette with the reagent;
- Adding the sample to the reagent, mixing and recording at least two absorbance values of the reaction mixture, the absorbance being measured at 630 nm;
- Recording two or more absorbance values of reaction mixture using the standard in place of the sample;
- Calculating the sample TAC as a function of the measured absorbances of the sample and of the standard and of the standard concentration.

## Patentansprüche

**1.** Kit zur photometrischen Bestimmung der gesamten antioxidativen Kapazität innerhalb und außerhalb von Zellen von Vollblut oder einer anderen biologischen Probe, umfassend:

- ein Reagenz, beinhaltend:

a) ein Chromogen, das empfindlich gegenüber dem elektrochemischen Potential der Probe ist, der Familie der Indophenole angehört, mit der allgemeinen Formel:

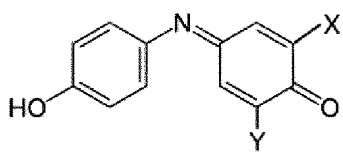

in der die Gruppen X und Y Halogenatome sind;
b) ein Puffersystem, um einen konstanten pH-Wert zwischen 8,8 und 9 zu halten;
c) ein Tensid mit zytolytischer Aktivität, bestehend aus Hydroxypolyethoxydodecan;
d) einen Chelatbildner;
e) eine Substanz, die die Polarität des Lösungsmittels verringern kann;
f) ein Konservierungsmittel;

- einen Standard, beinhaltend:

g) ein Puffersystem, um einen konstanten pH-Wert zu halten;
h) eine reduzierende Substanz;
i) einen Chelatbildner;
l) ein Konservierungsmittel.

**2.** Kit nach Anspruch 1, wobei das Chromogen (a) 2,6-Dichlorphenolindophenol oder 2,6-Dibromphenolindophenol oder eine Kombination davon ist.

**3.** Kit nach Anspruch 1 oder 2, wobei der Standard eine reduzierende Substanz (h), bestehend aus reduziertem Glutathion mit einer Konzentration im Bereich von 0,1 bis 10 mM, vorzugsweise mit einer Konzentration von 2 mM, und einen Stabilisator, bestehend aus Mannitol, umfasst.

**4.** Kit nach Anspruch 1, 2 oder 3, wobei das Puffersystem im Reagenz eine Lösung ist, die $H_2PO_4^-$/$HPO_4^{2-}$-Ionen beinhaltet, deren Konzentration zwischen 5 und 200 mM, vorzugsweise zwischen 50 und 100 mM, umfasst ist.

**5.** Kit nach Anspruch 1, 2 oder 3, wobei das Puffersystem im Standard eine Lösung ist, die $H_2PO_4^-$/$HPO_4^{2-}$-Ionen beinhaltet, deren Konzentration zwischen 5 und 200 mM, vorzugsweise zwischen 50 und 100 mM, umfasst ist und einen pH zwischen 5,0 und 6,5 aufweist.

**6.** Kit nach einem der vorstehenden Ansprüche, wobei der Chelatbildner Natriumethylendiamintetraacetat ($Na_2$EDTA) ist.

**7.** Kit nach einem der vorstehenden Ansprüche, wobei das Reagenz eine Substanz (e) umfasst, die die Polarität des Lösungsmittels verringern kann, wobei diese Substanz Ethylether, Methanol, Propanol oder Ethanol mit einer Konzentration im Bereich zwischen 5 % und 50 % (v/v), vorzugsweise Ethanol zwischen 10 % und 20 % (v/v) ist.

**8.** Kit nach einem der vorstehenden Ansprüche, wobei das Konservierungsmittel eine biozide Substanz ist, ausgewählt aus einer beliebigen Verbindung, die in Diagnosekits als Konservierungsmittel verwendet wird, vorzugsweise Methylthioazolinon oder Chloracetamid.

**9.** Methode zur Bestimmung der gesamten antioxidativen Kapazität (TAC) innerhalb und außerhalb von Zellen von Vollblut oder einer anderen biologischen Probe unter Verwendung eines Kits nach den Ansprüchen 1 bis 8 und eines Photometers, umfassend die Schritte des:

- Befüllens der Photometerküvette mit dem Reagenz;
- Zugebens der Probe zum Reagenz, Mischens und Erfassens von mindestens zwei Extinktionswerten der Reaktionsmischung, wobei die Extinktion bei 630 nm gemessen wird;
- Erfassens von zwei oder mehr Extinktionswerten der Reaktionsmischung unter Verwendung des Standards anstelle der Probe;
- Berechnens der Proben-TAC als eine Funktion der gemessenen Extinktionen der Probe und des Standards und der Standardkonzentration.

## Revendications

**1.** Kit de détermination photométrique de la capacité antioxydante totale à l'intérieur et à l'extérieur de cellules de sang total ou d'un autre échantillon biologique, comprenant :

- un réactif contenant :

a) un chromogène sensible au potentiel électrochimique de l'échantillon, appartenant à la famille des indophénols, ayant la formule générale :

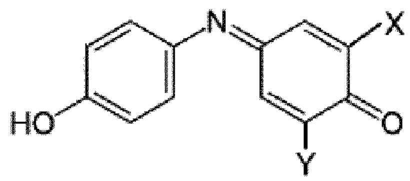

dans laquelle les groupes X et Y sont des atomes d'halogène ;
b) un système tampon, pour maintenir une valeur de pH constante comprise entre 8,8 et 9;
c) un tensioactif à activité cytolytique constitué d'hydroxypolyéthoxydodécane ;
d) un agent chélatant ;
e) une substance capable de réduire la polarité du solvant ;
f) un agent de conservation ;

- un étalon contenant :

g) un système tampon, pour maintenir une valeur de pH constante ;
h) une substance réductrice ;
i) un agent chélatant ;
l) un agent de conservation.

**2.** Kit selon la revendication 1, dans lequel le chromogène (a) est le 2,6-dichlorophénolindophénol ou le 2,6-dibromophénolindophénol ou une combinaison de ceux-ci.

**3.** Kit selon la revendication 1 ou 2, dans lequel l'étalon comprend une substance réductrice (h) constituée de glutathion réduit à une concentration allant de 0,1 à 10 mM, de préférence à une concentration de 2 mM, et un agent stabilisant constitué de mannitol.

**4.** Kit selon la revendication 1, 2 ou 3, dans lequel le système tampon dans le réactif est une solution contenant des ions $H_2PO_4^-/HPO_4^{2-}$ dont la concentration est comprise entre 5 et 200 mM, de préférence entre 50 et 100 mM.

**5.** Kit selon la revendication 1, 2 ou 3, dans lequel le système tampon de l'étalon est une solution contenant des ions $H_2PO_4$-/$HPO_4^{2-}$ dont la concentration est comprise entre 5 et 200 mM, de préférence entre 50 et 100 mM et ayant un pH entre 5,0 et 6,5.

**6.** Kit selon l'une quelconque des revendications précédentes, dans lequel l'agent chélatant est l'acide éthylène diamine tétra acétique de sodium ($Na_2$EDTA).

**7.** Kit selon l'une quelconque des revendications précédentes, dans lequel le réactif comprend une substance (e) capable de réduire la polarité du solvant, cette substance étant l'éther éthylique, le méthanol, le propanol ou l'éthanol à une concentration comprise entre 5 % et 50 % (v/v), de préférence l'éthanol entre 10 % et 20 % (v/v).

**8.** Kit selon l'une quelconque des revendications précédentes, dans lequel l'agent de conservation est une substance biocide, choisie parmi tout composé utilisé comme conservateur dans les kits de diagnostic, de préférence la méthylthioazolinone ou le chloroacétamide.

**9.** Procédé de détermination de la capacité antioxydante totale (TAC) à l'intérieur et à l'extérieur de cellules, de sang total ou d'un autre échantillon biologique, en utilisant un kit selon les revendications 1 à 8 et un photomètre, comprenant les étapes de :

- remplir la cuve de photomètre avec le réactif ;
- ajouter l'échantillon au réactif, mélanger et enregistrer au moins deux valeurs d'absorbance du mélange réactionnel, l'absorbance étant mesurée à 630 nm ;
- enregistrer deux valeurs d'absorbance ou plus du mélange réactionnel en utilisant l'étalon à la place de l'échantillon ;
- calculer la TAC de l'échantillon en fonction des absorbances mesurées de l'échantillon et de l'étalon et de la concentration de l'étalon.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GARRY et al.** *Clin. Biochem.,* 1974, vol. 7, 131-145
- **OMAYE et al.** *Meth. Enzymol.,* 1979, vol. 62, 3-11
- **HUANG et al.** *J. Agricult. Food Chem.,* 2005, vol. 53, 1841-1856
- **AKERBOOM, T.P. ; SIES, H.** Assay of glutathione, glutathione disulfide, and glutathione mixed disulfides in biological samples. *Methods Enzymol.,* 1981, vol. 77, 373-382
- **AKERBOOM, T.P. ; SIES, H.** Assay of glutathione, glutathione disulfide, and glutathione mixed disulfides in biological samples. *Methods Enzymol,* 1981, vol. 77, 373-382